Europäisches Patentamt

European Patent Office

Office européen des brevets

(19)

(11) EP 1 247 486 A2

(12) EUROPEAN PATENT APPLICATION

(43) Date of publication:
09.10.2002 Bulletin 2002/41

(51) Int Cl.⁷: A61B 5/022

(21) Application number: 02005061.3

(22) Date of filing: 06.03.2002

(84) Designated Contracting States:
AT BE CH CY DE DK ES FI FR GB GR IE IT LI LU
MC NL PT SE TR
Designated Extension States:
AL LT LV MK RO SI

(30) Priority: 30.03.2001 JP 2001098025

(71) Applicant: Omron Corporation
Kyoto, Kyoto 600-8530 (JP)

(72) Inventors:
• Kato, Hiroyuki, c/o Omron Corp., 801
-dori, Shimogyo-ku, Kyoto 600-8530 (JP)
• Tanaka, Takahide, c/o Omron Corp., 801
dori, Shimogyo-ku, Kyoto 600-8530 (JP)

• Sano, Yoshihiko,c/o Omron Corp., 801
dori, Shimogyo-ku, Kyoto 600-8530 (JP)
• Oku, Shojiro, c/o Omron Corp., 801
dori, Shimogyo-ku, Kyoto 600-8530 (JP)
• Kojima, Iwao, c/o Omron Corp., 801
dori, Shimogyo-ku, Kyoto 600-8530 (JP)
• Sawanoi, Yukiya,c/o Omron Corp., 801
dori, Shimogyo-ku, Kyoto 600-8530 (JP)

(74) Representative: Kilian, Helmut, Dr.
Wilhelms, Kilian & Partner
Patentanwälte
Eduard-Schmid-Strasse 2
81541 München (DE)

(54) **Electronic blood pressure monitor**

(57) To provide an electronic blood pressure monitor with increased measurement accuracy at the time of measurement of blood pressure at the wrist. A cuff has a pressure-increase structure for making a pressure to an ulna side of the wrist higher than a pressure to a radius side. The pressure-increase structure is achieved by providing an extrusion having a width narrower than a width of the cuff on the side facing the ulna portion. The extrusion is projected from the surface of the cuff. When the cuff is mounted on the wrist, the extrusion presses the ulnar portion with a pressure higher than an internal pressure of the cuff.

[Fig. 10]

EP 1 247 486 A2

## Description

BACKGROUND OF THE INVENTION

Field of the Invention

The present invention relates to an electronic blood pressure monitor for measuring a blood pressure at a wrist.

Description of the Background Art

An electronic blood pressure monitor for measuring a blood pressure at a wrist generally has a cuff to be wound around a wrist and a body provided integrally with or separately from the cuff. The cuff is provided with a pulse wave sensor for extracting pulse wave data of a region of an organism, and the body is provided with a display unit for displaying a blood pressure value and the like, and an operation unit for instructing start of measurement and the like.

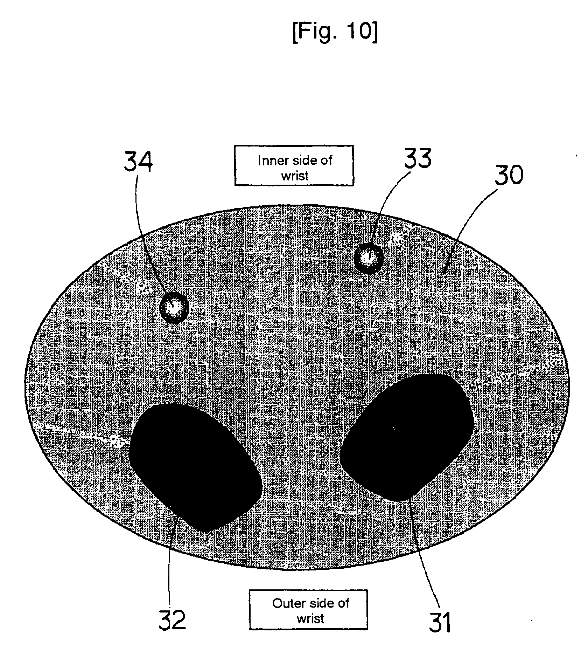

As shown in Fig. 10, in a wrist 30, the radius 31, ulna 32, radial artery 33, and ulnar artery 34 exist. The depth at which the radial artery 33 exists from the surface of an organism at the wrist 30 and that of the ulnar artery 34 at the wrist 30 are different from each other . Generally, the radial artery 33 is positioned closer to the surface of the wrist 30 than the ulnar artery 34.

Accordingly, the cuff pressure obtained by checking the blood flow in the radial artery 33 and that in the ulnar artery 34 are different from each other. The blood flow in the radial artery 33 positioned closer to the surface of the wrist 30 is checked at the cuff pressure lower than that of the ulnar artery 34.

A conventional wrist blood pressure monitor presses the radial artery 33 and the ulnar artery 34 simultaneously with a cuff wound around the wrist 30 and obtains a pulse wave signal. Consequently, when attention is paid to the radial artery 33, even if the cuff pressure achieves the cuff pressure obtained at which the blood flow in the radial artery 33 is completely checked (i.e., the highest blood pressure), there is a case that the blood flow in the ulnar artery 34 is not checked yet. In order to completely check the blood flow in the ulnar artery 34 as well, the cuff pressure has to be further increased.

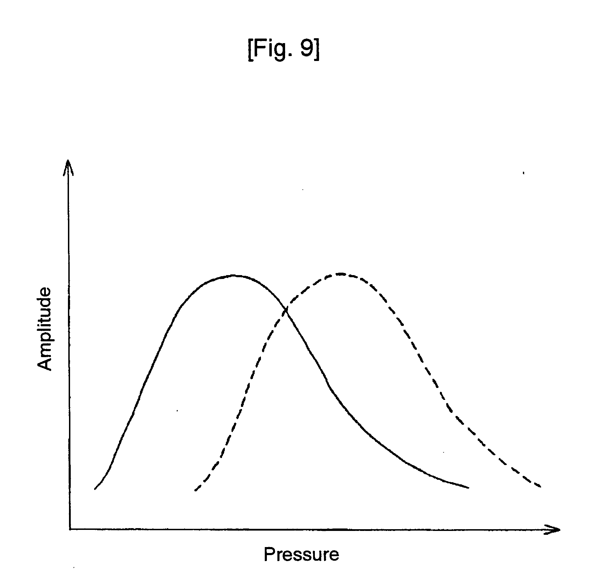

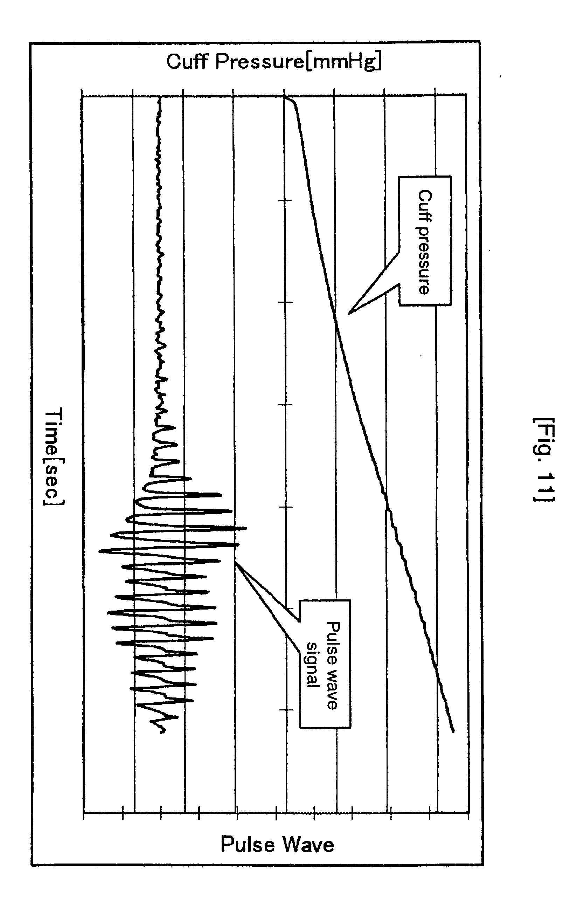

Since the cuff of the conventional wrist blood pressure monitor is a pressurizing unit having the same air line across the whole unit, the pressure of a pressing face attached to the wrist 30 on the radius side and that on the ulna side are the same. As the depths and structures of the radial artery 33 and ulnar artery 34 are different from each other, the pressure to the radial artery 33 and that to the ulnar artery 34 are different from each other. Consequently, pulse wave data obtained by the pulse wave sensor is combination of pulse wave data from the radial artery 33 and pulse wave data from the ulnar artery 34. More specifically, as shown in Fig. 11 (graph showing the correlation of time, cuff pressure, and pulse wave signal), an envelope of a pulse wave signal obtained by the cuff for the conventional wrist blood pressure monitor often has two peaks (refer to Fig. 9) such that a pulse wave signal from the radial artery 33 appears first and a pulse wave signal from the ulnar artery 34 appears next. Due to this, it has been difficult to determine the blood pressure accurately.

SUMMARY OF THE INVENTION

Therefore, the present invention has been achieved while paying attention to such a problem and provides an electronic blood pressure monitor with increased measurement accuracy in measurement at the wrist.

An electronic blood pressure monitor of the present invention includes: a pressurizing unit to be mounted on a wrist of a subject for applying pressure to the wrist; a pressure-increase structure for applying to a portion of the wrist above ulna a pressure higher than a pressure applied to a portion of the wrist above radius, the structure being connected to the pressurizing unit; a pulse wave detecting device for extracting pulse wave data from measured pressure data of the pressurizing unit; a measuring device for measuring blood circulation dynamics using the pulse wave data; and a display unit for displaying a result of measurement of the blood circulation dynamics .

In the electronic blood pressure monitor, when the pressurizing unit is mounted on the wrist, the ulna portion is pressed by the pressure-increase structure with a pressure higher than a pressure applied to the radial portion. As described above, although the ulnar artery is positioned on the inner side as compared with the radial artery from the surface of an organism, since the ulna portion is pressed with a stronger force, the pressure to the radial artery and that to the ulnar artery can be made substantially the same, and the blood flow in both of the radial and ulnar arteries is checked. As a result, at the time of measuring the blood pressure, unlike the conventional technique, only pulse wave data from the radial artery can be extracted, so that the blood pressure can be accurately measured.

A pressure-increase structure for applying a pressure higher than the above-described pressure to the ulna portion may be also provided. By pressing the ulnar artery before the radial artery is pressed, the blood flow in the ulnar artery is checked before the blood flow in the radial artery is checked. As a result, similarly, at the time of measuring the blood pressure, unlike the conventional technique, only pulse wave data from the radial artery can be extracted, so that the blood pressure can be accurately measured. Particularly, when it is constructed so that the ulnar artery is completely pressed at the lowest blood pressure or less (closed state) , during measurement from the highest blood pressure to the lowest blood pressure, the pulse wave data only from the radial artery can be accurately obtained. Thus, the blood pressure can be measured more accurately as compared with the above.

The pressure-increase structure in the electronic blood pressure monitor has an extrusion provided on the pressurizing unit so that the extrusion is placed over ulna when the pressurizing unit is mounted on the wrist. The extrusion has a width narrower than a width of the pressurizing unit. The extrusion may be made of a non-compressible material or may be an air bag connected to an air line different from an air line connected to the pressurizing unit. When sandwiched between the pressurizing unit and the wrist, the non-compressible material is not so compressed as air but has moderate hardness to sufficiently closes the ulnar artery existing in the wrist. Substances corresponding to the material are gel, a polymer of high molecule compound, rubber, solution, liquid closed in a bag, sponge and the like.

An electronic blood pressure monitor of another embodiment of the invention includes: a pressurizing unit to be mounted on a wrist of a subject for applying pressure to the wrist; a vibration absorption structure provided on or in the pressurizing unit for absorbing wave pulse vibration at a portion of the wrist above ulna; a pulse wave detecting device for extracting pulse wave data from measured pressure data of the pressurizing unit; a measuring device for measuring blood circulation dynamics using the pulse wave data; and a display unit for displaying a result of measurement of the blood circulation dynamics.

In the electronic blood pressure monitor, when the pressurizing unit is mounted on the wrist, pulse wave vibration from the ulnar artery is absorbed by the vibration absorption structure. More specifically, even in a state where although the blood flow in the radial artery is checked, the blood flow in the ulnar artery is not completely checked, the pulse wave vibration from the ulnar artery is absorbed by the vibration absorption structure. As a result, only the pulse wave data of the radial artery can be extracted and, similarly, the blood pressure can be measured accurately.

The vibration absorption structure in the electronic blood pressure monitor has, for example, a vibration absorption material provided on or in the pressuring unit so that the absorption structure is placed over ulna when the pressuring unit is mounted on the wrist. An example of the vibration absorbing material is a shock absorbing gel, and the shock absorbing gel is made of, for example, a polymer of high molecule compound.

In the present invention, blood circulation dynamics measured by the measuring device denotes pressure (the highest blood pressure and the lowest blood pressure) or a blood vessel state.

BRIEF DESCRIPTION OF THE DRAWINGS

Fig. 1 is a perspective view of an electronic blood pressure monitor according to an embodiment;
Figs. 2A and 2B are partially omitted plan view and a partially omitted side view, respectively, showing an example of the form of a cuff having a pressure-increase structure in the electronic blood pressure monitor;
Figs. 3A and 3B are partially omitted plan view and a partially omitted side view, respectively, showing another example of the form of a cuff having a vibration absorption structure in the electronic blood pressure monitor, and Fig. 3C is a partially omitted side view showing a modification of the cuff;
Fig. 4 is a block diagram showing a general schematic configuration of the electronic blood pressure monitor;
Fig. 5 is a schematic sectional view showing a state where the cuff of Figs. 2A and 2B is mounted on the wrist;
Fig. 6 is a schematic sectional view showing a state where the cuff of Figs. 3A and 3B is mounted on the wrist;
Fig. 7 is a diagram for explaining a pressing action by an extrusion when the cuff of Figs. 2A and 2B is mounted on the wrist;
Fig. 8 is a graph showing the correlation of time, cuff pressure and pulse wave signal obtained when the cuff of Figs. 2A and 2B or Figs. 3A or 3B is used;
Fig. 9 is a diagram showing a pulse wave signal obtained when a cuff of a conventional electronic blood pressure monitor is used;
Fig. 10 is a schematic sectional view showing the inside of a wrist; and
Fig. 11 is a graph showing correlation of time, cuff pressure and pulse wave signal obtained when the cuff of the conventional electronic blood pressure monitor is used.

DETAILED DESCRIPTION OF THE PREFERRED EMBODIMENT

Embodiments of the invention will be described hereinafter.

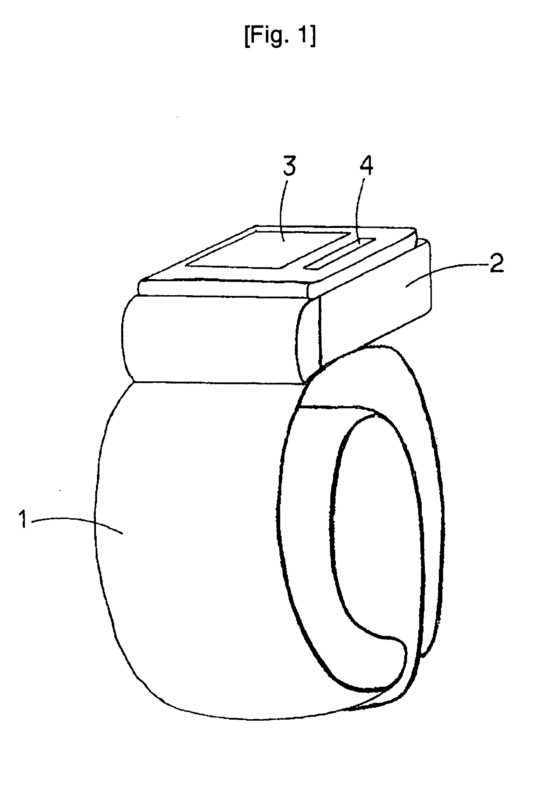

Fig. 1 is a perspective view of an electronic blood pressure monitor according to an embodiment of the present invention. The electronic blood pressure monitor measures a blood pressure at the wrist, and includes a pressurizing unit (cuff) 1 mounted on the wrist, and a body 2 integrally or detachably provided for the cuff 1. The cuff 1 has an air bag inflated/deflated by charging/discharging air, a curler for holding the shape, and a pulse wave sensor for extracting pulse wave data at the wrist. The body 2 has a display unit 3 for displaying a blood pressure value, abnormal measurement, and the like, and an operation unit 4 for instructing start of measurement and the like.

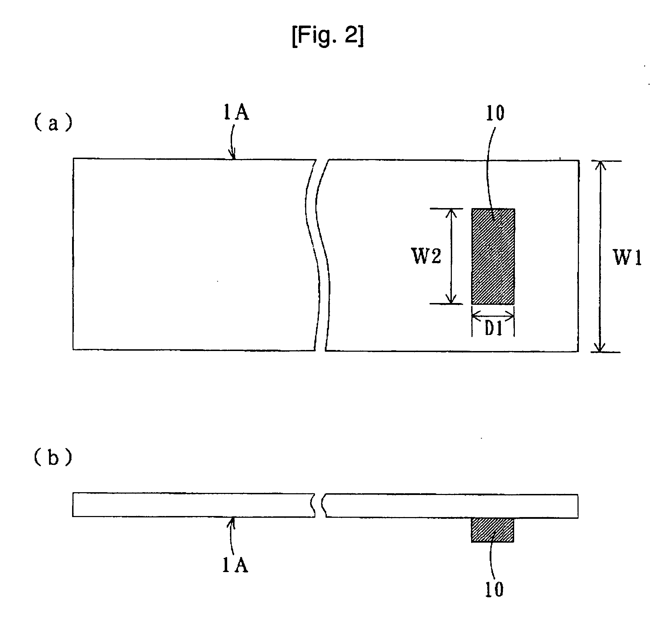

Figs. 2A and 2B are a partially omitted plan view and a partially omitted side view, respectively, of an example of the shape of the cuff 1 in the electronic blood pressure monitor. This cuff 1A has a pressure-increase structure for making the pressure applied to the ulna side of the wrist higher than that applied to the radius side. In the pressure-increase structure, an extrusion 10 having a width narrower than the width of the pressurizing unit is provided on the side corresponding to the ulnar portion. The extrusion 10 is positioned near to one end of the cuff 1A, projected from the surf ace of the cuff 1A, and positioned so as to face the ulnar portion when the cuff 1A is mounted on the wrist.

A width W2 of the extrusion 10 is set to about the half of a width W1 of the cuff 1A. Concretely, the width W2 of the extrusion 10 is 30 to 40 mm, and a length D1 of the extrusion 10 is 10 to 20 mm. When it is assumed that the hardness of the cuff 1A is 100 mm/kg, the hardness of the extrusion 10 is 200 mm/kg. The extrusion may be a non-compressible material or an air bag connected to an air line different from an air line connected to the pressurizing unit. When sandwiched between the pressurizing unit and the wrist, the non-compressible material is not so compressed as air but has moderate hardness to sufficiently closes the ulnar artery existing in the wrist. Substances corresponding to the material are gel, polymer of high molecule compound, rubber, solution, liquid closed in a bag, sponge and the like.

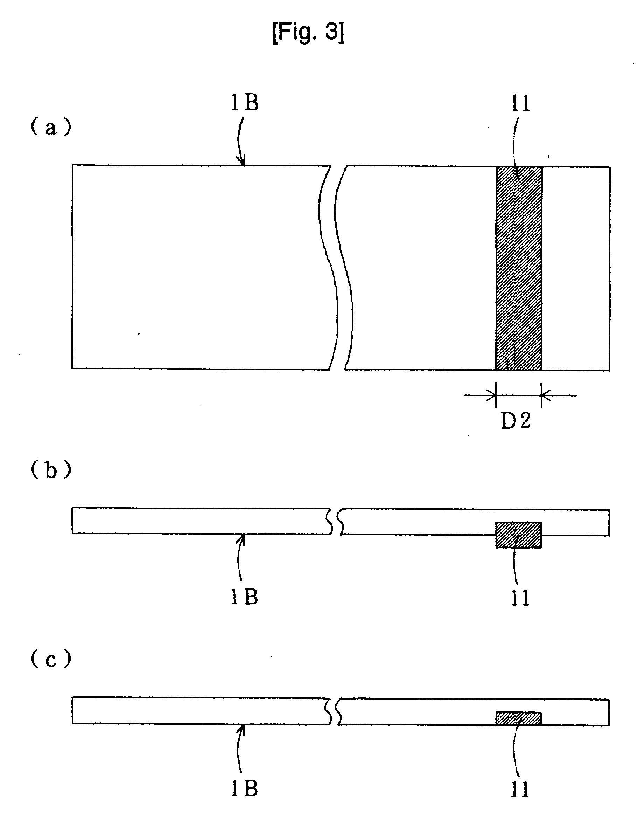

Figs. 3A and 3B are a partially omitted plan view and a partially omitted side view, respectively, showing another example of the form of the cuff 1 in the electronic blood pressure monitor. This cuff 1B has a vibration absorption structure for absorbing pulse wave vibration on the ulna side of the wrist. In this case, the vibration absorption structure is achieved by providing a vibration absorption material 11 on the side facing the ulna side. The vibration absorption material 11 is a shock absorbing gelmadeof, for example, a polymer of high molecule compound. The vibration absorbing material 11 is provided over the full width in a position near to one end of the cuff 1B and is slightly projected from the surface of the cuff 1B. The length D2 of the vibration absorption material 11 is the same as the length D1 of the extrusion 10, which is 10 to 20 mm.

The vibration absorption material 11 does not have to be protruded from the surface of the cuff 1B and, as shown in Fig. 3C, the surface of the vibration absorption material 11 and that of the cuff 1B may be approximately flush with each other.

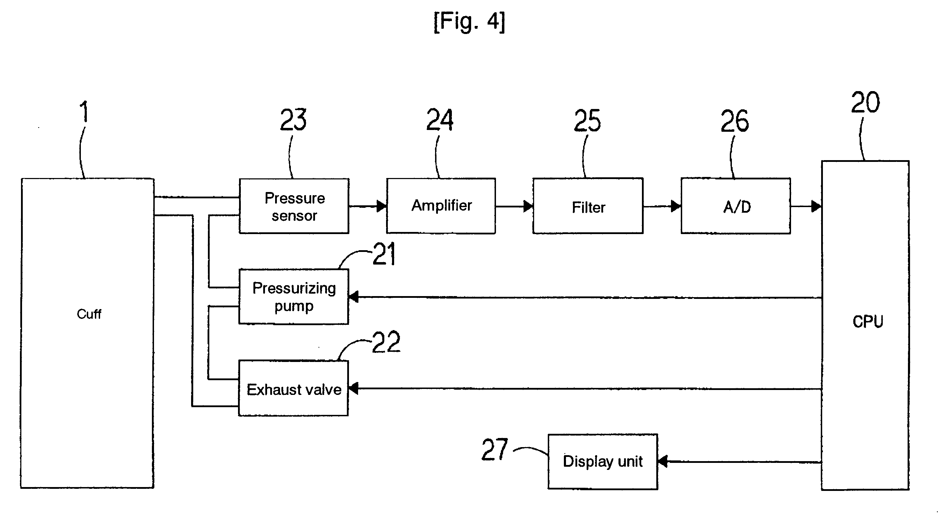

Fig. 4 is a block diagram showing the general configuration of the electronic blood pressure monitor according to the embodiment. The electronic blood pressure monitor has a cuff 1 (the cuff 1A or 1B) mounted on the wrist to press the wrist, a pressurizing pump 21 for increasing the pressure in the cuff 1, an exhaust valve (pressure control unit) 22 for decreasing the pressure in the cuff 1, a pressure sensor (pressure detecting unit) 23 for detecting the pressure in the cuff 1, an amplifier 24 for amplifying an output from the pressure sensor 23, a filter 25 for extracting only a pulse wave component included in a cuff pressure signal as the output of the amplifier 24, an A/D converter 26 for converting analog signals from the amplifier 24 and filter 25 into digital signals and inputting the digital signals to a CPU 20, a display unit 27 for displaying a calculated blood pressure value and the like, and the CPU 20 for controlling the pressurizing pump 21, exhaust valve 22, display unit 27 and the like.

The CPU 20 has a measuring function of measuring blood circulation dynamics (blood pressure and blood vessel state) on the basis of pulse wave data detected by the pressure sensor 23. That is, the CPU 20 applies a predetermined algorithm to the cuff pressure signal captured and determines the highest and lowest blood pressures.

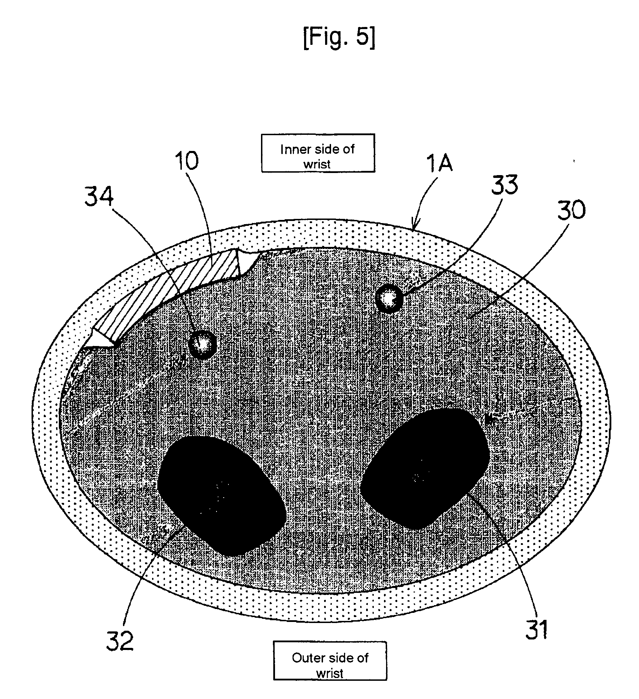

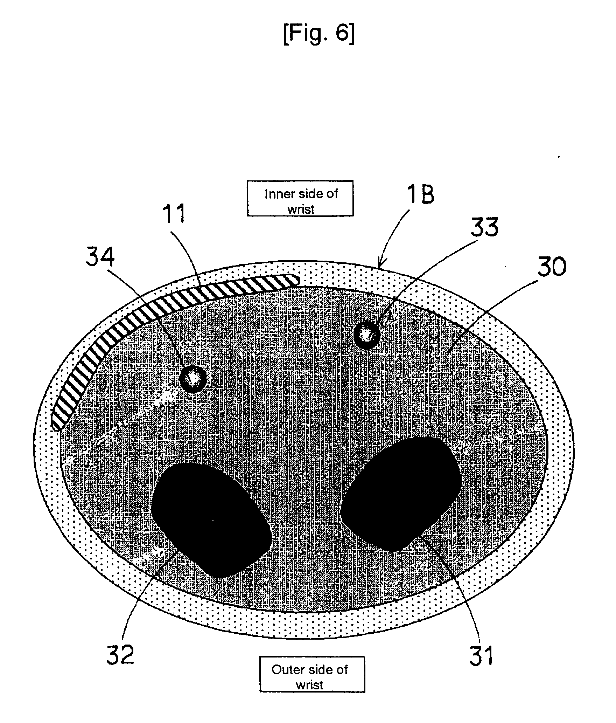

The configuration described above is similar to that of the conventional blood pressure monitor. The electronic blood pressure monitor according to the embodiment has a feature that the cuff 1 is the cuff 1A or 1B of the above structure. Figs. 5 and 6 show states where the cuffs 1A and 1B are mounted on the wrist.

Fig. 5 is a schematic sectional view showing a state where the cuff 1A is mounted on the wrist 30. In the mounting state of the cuff 1A, the extrusion 10 faces the ulnar portion of the wrist 30 and the ulnar portion is pressed by the moderately hard extrusion 10. Even if the internal pressure is uniform in the cuff 1A, the ulnar portion is pressed harder by the extrusion 10. Consequently, even when the ulnar artery 34 is positioned on the inner side as compared with the radial artery 33 from the surface of an organism, the radial artery 33 and ulnar artery 34 are pressed with approximately same pressure, and the blood flow in both of the arteries is checked. As a result, at the time of measuring the blood pressure, only a signal from the radial artery 33 can be selectively extracted, and a signal from the ulnar artery 34 is not mixed, so that accurate blood pressure measurement can be achieved.

On the other hand, as shown in Fig. 6, when the cuff 1B is mounted on the wrist 30, the vibration absorption material 11 faces the ulnar portion. The pressure to the radial artery 33 and that to the ulnar artery 34 by the cuff 1B are approximately same. Although the blood flow in the radial artery 33 is checked, the blood flow in the ulnar artery 34 is not completely checked. However, pulse wave vibration from the ulnar artery 34 is absorbed by the vibration absorption material 11. Consequently, the pulse wave signal from the ulnar artery 34 is not detected by the pressure sensor 23, and only the pulse wave signal from the radial artery 33 is detected by the pressure sensor 23. As a result, only the signal of the radial artery 33 can be extracted and, similarly, accurate blood pressure measurement can be performed.

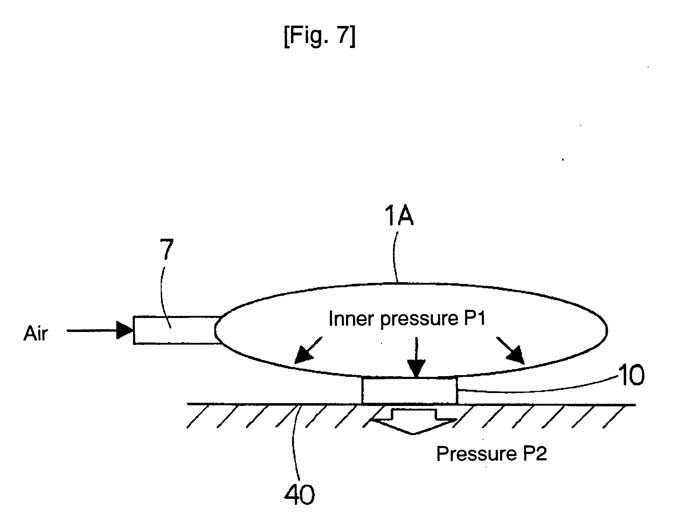

The pressing action of the extrusion 10 when the cuff 1A is mounted on the wrist 30 will now be described with reference to Fig. 7. As described above, the width W2 of the extrusion 10 is narrower than the width W1 of the cuff 1A (refer to Fig. 2A), a pressure P2 higher than an internal pressure P1 of the cuff 1A (equal to the pressure on the radius portion) is applied to the ulnar side. The principle is as follows.

In Fig. 7, when air is supplied to the cuff 1A from an air supply port 7, the air bag of the cuff 1A is inflated, and an internal pressure P1 is increases in the cuff 1A. The internal pressure P1 is applied to an organism pressing face 40 via the extrusion 10, and the extrusion 10 presses the organism pressing face 40 with the pressure P2. At this time, the pressure P2 is higher than the internal pressure P1.

When the cross sectional area parallel to the organism pressing face 40 of the cuff 1A is S1 and the cross sectional area parallel to the organism pressing face 40 of the extrusion 10 is S2, the pressure P2 is calculated by the following equation.

$$P2 = (S1/S2) \times P1$$

More specifically, for example, when S1/S2 is 2/1, the pressure P2 twice as high as the internal pressure P1 is applied to the organism pressing face 40.

By setting the width W2 of the extrusion 10 to a fraction (for example, 1/2) of the width W1 of the cuff 1A by using the principle, the pressure of W1/W2 is applied to the ulnar portion. Therefore, by setting the widths W1 and W2 so that the ulnar artery 34 is closed at a usual lowest blood pressure (for example, 50 mm-Hg) or less, the ulnar artery 34 is closed at a lower cuff pressure as compared with the radial artery 33. As a result, at the time of measuring the blood pressure, only the signal from the radial artery 33 can be selectively extracted, and higher-precision blood pressure measurement can be therefore performed.

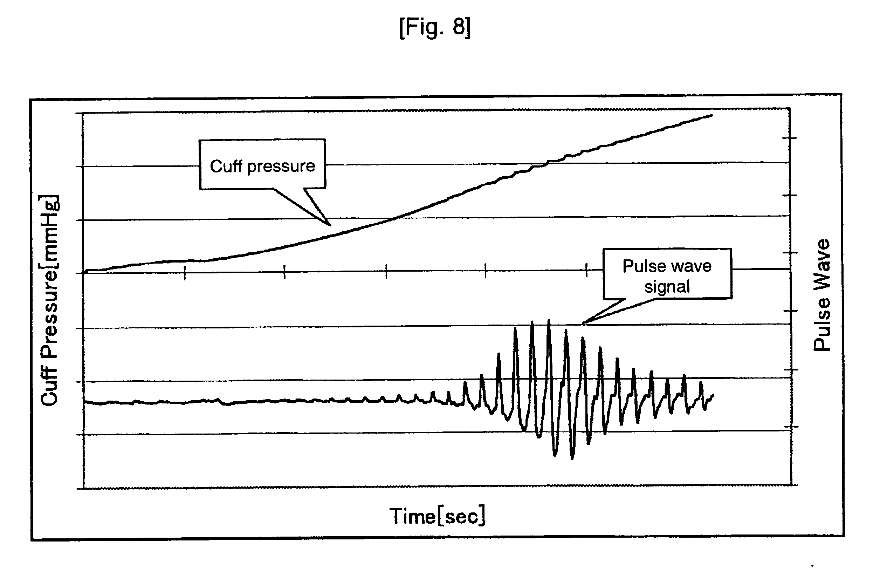

As described above, by using the cuff 1A or 1B, the accurate blood pressure can be measured. As shown in Fig. 8 (a graph showing the correlation of time, cuff pressure and pulse wave signal), the pulse wave signal obtained by using the cuff 1A or 1B is only a pulse wave signal from the radial artery unlike the conventional case (Fig. 11) having two peaks in which the pulse wave signal from the ulnar artery is mixed.

In other words, conventionally, in Fig. 9, the pulse wave signal (broken line) from the ulnar artery appears subsequent to the pulse wave signal (solid line) from the radial artery at the time of measuring the blood pressure. By using the cuff 1A or 1B, the pulse wave signal from the ulnar artery does not appear.

As described above, the electronic blood pressure monitor according to the present invention including either the pressurizing unit having the pressure-increase structure or the vibration absorption structure can extract only the pulse wave data from the radial artery at the time of measuring the blood pressure, so that the blood pressure can be accurately measured.

**Claims**

1. An electronic blood pressure monitor comprising:

   a pressurizing unit to be mounted on a wrist of a subject for applying pressure to the wrist;
   a pressure-increase structure for applying to a portion of the wrist above ulna a pressure higher than a pressure applied to a portion of the wrist above radius, the structure being connected to the pressurizing unit;
   a pulse wave detecting device for extracting pulse wave data from measured pressure data of the pressurizing unit;
   a measuring device for measuring blood circulation dynamics using the pulse wave data ; and
   a display unit for displaying a result of measurement of the blood circulation dynamics.

2. The electronic blood pressure monitor of claim 1, wherein the pressure-increase structure comprises an extrusion provided on the pressurizing unit so that the extrusion is placed over ulna when the pressurizing unit is mounted on the wrist, the extrusion having a width narrower than a width of the pressurizing unit.

3. The electronic blood pressure monitor of claim 2, wherein the extrusion is made of a non-compressible material.

4. The electronic blood pressure monitor of claim 2, wherein the extrusion comprises an air bag connected to an air line different from an air line connected to the pressurizing unit.

5. An electronic blood pressure monitor comprising:

   a pressurizing unit to be mounted on a wrist of a subject for applying pressure to the wrist;
   a vibration absorption structure provided on or in the pressurizing unit for absorbing wave pulse vibration at a portion of the wrist above ulna;
   a pulse wave detecting device for extracting pulse wave data from measured pressure data of the pressurizing unit;
   a measuring device for measuring blood circulation dynamics using the pulse wave data; and
   a display unit for displaying a result of measurement of the blood circulation dynamics.

6. The electronic blood pressure monitor of claim 5, wherein the absorption structure comprises a vibration absorption material provided on or in the pressurizing unit so that the absorption structure is placed over ulna when the pressurizing unit is mounted on the wrist.

7. The electronic blood pressure monitor of claim 6, wherein the vibration absorbing material comprises a shock absorbing gel.

8. The electronic blood pressure monitor of claim 7, wherein the sock absorbing gel is made of a polymer of high molecule compound.

9. An electronic blood pressure monitor comprising:

a pressurizing unit to be mounted on a wrist of a subject for applying pressure to the wrist; a pulse wave detecting device for extracting pulse wave data from measured pressure data of the pressurizing unit; a device for preventing a pulse wave generated by ulnaris from being detected by the pulse wave detecting device; a measuring device for measuring blood circulation dynamics using the pulse wave data; and a display unit for displaying a result of measurement of the blood circulation dynamics.

[Fig. 1]

[Fig. 2]

( a )

1 A          10

W2          W1

D1

( b )

1 A

10

[Fig. 3]

( a )

1B

11

D 2

( b )

1B

11

( c )

1B

11

[Fig. 4]

EP 1 247 486 A2

[Fig. 5]

Inner side of wrist

Outer side of wrist

[Fig. 6]

Inner side of wrist

Outer side of wrist

[Fig. 7]

[Fig. 8]

[Fig. 9]

[Fig. 10]

34     Inner side of wrist     33     30

32     Outer side of wrist     31

EP 1 247 486 A2

[Fig. 11]

17